# EUROPÄISCHE PATENTSCHRIFT

(11) **EP 0 630 657 B1**
(45) Veröffentlichungstag und Bekanntmachung des Hinweises auf die Patenterteilung: **28.07.1999**
(21) Anmeldenummer: 94108025.1
(22) Anmeldetag: 25.05.1994
(51) Int. Cl.: A61M 25/01, A61M 25/00

(54) **Katheter aus einem biegsamen Kunststoffschlauch**
Bendable plastic tube catheter
Cathéter de matière plastique pliable

(30) Priorität: 24.06.1993 DE 4320962
(43) Veröffentlichungstag der Anmeldung: 28.12.1994
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Schmitt, Hans, Dipl.-Ing.

(56) Entgegenhaltungen:
- EP-A- 0 489 937
- EP-A- 0 521 595
- EP-A- 0 543 539
- WO-A-92/14506
- DE-A- 3 920 707
- US-A- 5 041 085

## Beschreibung

Die Erfindung betrifft einen Katheter mit wenigstens einem in seinem Inneren verlaufenden hohlen Kanal oder Lumen gemäß dem Oberbegriff des Patentanspruches 1.

Ein derartiger Katheter ist aus EP-A-0 489 937 bereits bekannt. Dabei sieht eine Ausführungsform gemäß Fig.8 vor, daß als Bewehrung für das Zugelement ein als Hülse bezeichnetes Führungsrohr aus biegestabilem Material wie Metall oder hartem Kunststoff vorgesehen ist, das am Übergang zu der auslenkbaren Spitze endet. Das Zugelement läuft hingegen weiter bis zu der Spitze und bleibt dabei auf seinem Abstand zur Mittelachse des Katheters, so daß der wirksame Hebelarm für die Biegebewegung durch das Führungsrohr mitbestimmt und eingeschränkt wird.

Eine vergleichbare Anordnung ist aus DE-A-39 20 707 bekannt. In einem exzentrischen Bereich des Katheters verläuft in einem separaten Kanal ein Zugelement, wobei dieser exzentrische Kanal mit Abstand zu der Spitze endet, wo das Zugelement austritt, aber seinen Abstand beibehält.

Aus WO-A-92/14506 ist ebenfalls ein derartiger Katheter bekannt. Dabei verläuft das Zugelement hinter seinem Austritt aus einem Führungsrohr geradlinig weiter, behält also ebenfalls den schon von dem Führungsrohr vorgegebenen Abstand zur Längsmittelachse des Katheters.

Der Erfindung liegt die Aufgabe zugrunde, einen Katheter der gattungsgemäßen Art zu schaffen, bei welchem das Zugelement in dem auslenkbaren Bereich so weit wie möglich radial nach außen verlegt sein kann, um eine bestmögliche Krümmungswirkung ausüben zu können, obwohl das Zugelement bis zu dem auslenkbaren Bereich innerhalb eines Führungsrohres verläuft.

Diese Aufgabe wird bei einem gattungsgemäßen Katheter mit den Mitteln und Merkmalen des Patentanspruches 1 gelöst.

Durch den Übergangsbereich des Führungskanales wird das Zugelement in dem auslenkbaren Bereich so weit wie irgend möglich radial nach außen verlegt, um eine bestmögliche Krümmungswirkung auf diesen auslenkbaren Bereich ausüben zu können, wahrend innerhalb des Führungsrohres dessen Wandstärke zu einem etwas weiter innenliegenden Verlauf dieses Zugelementes führt, wo aber eine Auslenkung durch die Bewehrung unterbunden ist.

Dabei kann der Umriß des Querschnittes des Katheters stetig und vorsprungfrei sein. Dennoch wird beim Aktivieren eines Manipulators für das Zugelement der Katheter nur an der bewegbaren oder ausbiegbaren Spitze und nicht über seine gesamte Länge - und dies mit bestmöglicher Wirkung - gekrümmt.

Zweckmäßig ist es, wenn das Führungsrohr aus Metall, zum Beispiel aus Edelstahl, oder gegebenenfalls aus hartem Kunststoff besteht. Dadurch kann erreicht werden, daß lediglich der auslenkbare Bereich mit Hilfe des Zugelementes gebogen wird, weil in dem übrigen Bereich durch das zwar biegsame aber im wesentlichen widerstandsfähige Führungsrohr der von diesem bewehrte Bereich des Katheters an einer Biegung gehindert wird. Dies rührt daher, daß der auslenkbare Bereich aufgrund seiner Weichheit wesentlich leichter als der mit dem Führungsrohr versehene Bereich gebogen werden kann. Vorteilhaft ist dabei, daß das Zugelement innerhalb des Umrisses des auslenkbaren Bereiches radial möglichst weit nach außen verlegt bis zu seiner Befestigungsstelle am distalen Ende verläuft.

Das das Zugelement in sich aufnehmende Führungsrohr kann einen Außendurchmesser von etwa einem Drittel oder zwei Fünftel Millimeter bis ungefähr ein Millimeter und einen Innendurchmesser von etwa ein Fünftel bis etwa vier Fünftel Millimeter haben. Somit nimmt es innerhalb des Katheters so wenig Platz ein, daß es gut innerhalb des Umrisses des Katheters untergebracht werden kann und ein gegenüber dem Umriß außen vorstehender Wulst vermieden werden kann.

Der Querschnitt oder Durchmesser des Zugelementes, insbesondere eines Zugdrahtes, Fadens oder Bandes, kann mit einem Spiel von etwa einem Hundertstel bis etwa einem Zehntel Millimeter gegenüber dem Innenquerschnitt des Führungsrohres versehen sein. Dabei kann das Zugelement ein Metallfaden oder Metallband sein. Für die Aufnahme eines Bandes kann dabei das Führungsrohr abgeflacht sein, so daß es in radialer Richtung innerhalb des Katheters noch weniger Platz benötigt, wenn die längere Querschnittsachse dieses flachen Rohres und die größere breite des Querschnittes des Metallbandes etwa in Umfangsrichtung des Katheters angeordnet sind.

Der den Katheter bildende Kunststoffschlauch kann zumindest im Bereich der Anordnung des Führungsrohres für das Zugelement eine größere Wandstärke als in dem auf einem Durchmesser etwa gegenüberliegenden Wandbereich haben und das Führungsrohr kann wenigstens teilweise in dem verdickten Wandbereich eingebettet sein. Auf diese Weise kann seine aussteifende Wirkung gut auf die Katheterwandung übertragen werden. Dennoch ist das Führungsrohr mit dem Zugelement innerhalb des Außenumrisses des Katheters untergebracht.

In dem verdickten Wandbereich kann benachbart zu dem Führungsrohr wenigstens ein zusätzlich zu dem inneren Kanal paralleler weiterer Kanal mit insbesondere kleinerem Querschnitt verlaufen. Es kann also die Verdickung der Wand zu Unterbringung des Führungsrohres dazu ausgenutzt werden, wenigstens einen weiteren Kanal vorzusehen, so daß beispielsweise durch den Innenkanal größeren Querschnittes ein Instrument oder Endoskop oder dergleichen und durch diesen zweiten Kanal ein Medikament zugeführt werden können.

Eine abgewandelte Ausführung der Erfindung kann darin bestehen, daß der Katheter aus zwei ineinandersteckenden Kunststoffrohren oder Kunststoffschläuchen gebildet ist, wobei das äußere Rohr die dichte Außenhülle bildet, und daß das innere Rohr in seiner Längsrichtung mit Abstand zueinander Jeweils radial einwärts gerichtete Klemmfinger, Haltezungen oder Laschen zum Festlegen oder Einklemmen den innenseitig verlaufenden Führungsrohres für das Zugelement aufweist. In diesem Falle ist also das Führungsrohr nicht in eine verdickte Wandung des Katheters eingebettet, sondern von entsprechenden Laschen, Fingern oder dergleichen an der Innenseite gehalten.

Die Haltezungen oder dergleichen des inneren Rohres können zusätzlich zu dem insbesondere abgeflachten Führungsrohr ein weiteres Rohr als zusätzlichen Kanal halten. Somit ist auch bei dieser Ausführungsform zusätzlich zu dem Hauptkanal noch ein weiterer paralleler Kanal mit kleinerem Querschnitt möglich. Die Abflachung der Rohre bewirkt dabei eine möglichst geringe Querschnittsverengung des Innenkanales des Katheters, in welchen die Haltezungen und dergleichen für die Festlegung des Führungsrohres etwas hineinragen.

Eine abgewandelte Ausführung der erfindungsgemäßen Lösung kann darin bestehen, daß als eine zusätzliche Bewehrung parallel zu dem exzentrisch angeordneten Zugelement auf der etwa auf einem Durchmesser gegenüberliegenden Seite ein weiteres Zugelement fest installiert ist, welches bis zu dem Beginn des biegsamen Bereiches verläuft und dort befestigt ist. Wird nun eine Zugkraft auf das bewegbare Zugelement ausgeübt, trägt das ihm am Durchmesser in dem Katheter gegenüberliegende Zugelement dazu bei, eine Biegung oder Krümmung dieses Katheterbereiches zu verhindern. Ein Zugelement als zusätzliche Bewehrung hat den Vorteil, die Biegsamkeit des Katheters selbst, die bei seinem Einführen in Blutgefäße vorteilhaft ist, praktisch nicht zu beeinträchtigen.

Vor allem bei Verwendung eines Zugelementes als zusätzliche Bewehrung kann der den Katheter bildende Schlauch eine Wand etwa gleichbleibender Dicke haben und an sich auf einem Durchmesser gegenüberliegenden Seiten einerseits einen Kanal mit Führungsrohr für das zum biegen bewegbare Zugelement und andererseits einen Kanal für das unverschiebbare bis zu dem auslenkbaren Bereich führende und dort endende Zugelement insbesondere einen festen Draht oder ein Band zum Beispiel aus Metall, enthalten.

Auch bei dieser Anordnung kann parallel zu den Kanälen für Zugelement und Bewehrung wenigstens ein weiterer Kanal als Zusatzlumen in der Wandung des Katheters angeordnet sein. Somit kann auch bei dieser Anordnung zusätzlich zu dem zentralen oder Haupt-Kanal ein weiterer Kanal kleineren Innenquerschnittes untergebracht werden.

Vor allem bei Kombination einzelner oder mehrerer der vorbeschriebenen Merkmale und Maßnahmen wird erreicht, daß der Katheter eine genügende Flexibilität haben kann, um beispielsweise auch in Blutgefäße und durch deren Krümmungen eingeführt werden zu können, aber so weit ausgesteift ist, daß die Betätigung des Zugelementes nicht zu einer Krümmung des gesamten Katheters führt, sondern nur die auslenkbare und steuerbare Katheterspitze betrifft. Dennoch kann der Katheter einen stetigen nicht durch einen vorspringenden Wulst veränderten Querschnittsumfang haben und ein im auslenkbaren Bereich liegender Zugdraht mit einer Verletzungsgefahr wird vermieden.

Nachstehend sind Ausführungsbeispiele der Erfindung anhand der Zeichnung näher beschrieben. Es zeigt in zum Teil schematisierter Darstellung:
- Fig.1: einen erfindungsgemäßen steuerbaren Katheter mit auslenkbarem Bereich an seinem distalen Ende und mit einem durch Drehen betätigbaren Manipulator amproximalen Ende,
- Fig.2: in vergrößertem Maßstab die Einzelheit in dem Kreis A in Figur 1, wobei am distalen auslenkbaren Bereich Elektroden zur Aufnahme intrakardialer Signale angedeutet sind,
- Fig.3: in vergrößertem Maßstab einen Längsschnitt durch den Teil des Katheters, an welchem der auslenkbare Bereich beginnt gemäß der Einzelheit B in Figur 1,
- Fig.4: einen Querschnitt gemäß der Linie IV-IV in Figur 3,
- Fig.5: in perspektivischer Darstellung einen Abschnitt des Katheters mit Sicht auf seinen Querschnitt etwa im Bereich der Schnittlinie IV-IV in Figur 3, wobei zusätzliche Kanäle neben dem als Bewehrung dienenden Führungsrohr zur Aufnahme des Zugelementes vorgesehen sind,
- Fig.6: eine der Figur 5 entsprechende Darstellung einer abgewandelten Ausführungsform, bei welcher als zusätzliche Bewehrung parallel zu dem Führungsrohr für das bewegbare Zugelement ein weiteres festverankertes Zugteil an einem Durchmesser gegenüberliegend angeordnet ist,
- Fig.7: eine abgewandelte Ausführungsform des Katheters mit in seinem Inneren angeordnetem Bewehrungs- und Führungsrohr zur Aufnahme des Zugelementes, wobei der Katheter aus zwei Rohren oder Schläuchen besteht, deren Innerer Haltelaschen für das Führungsrohr hat,
- Fig.8: eine der Figur 7 etwa entsprechende Ausführungsform, bei welcher die Laschen des inneren Katheterschlauches oder -rohres zwei im Querschnitt abgeflachte Rohre halten, deren eines ein Zugelement aufnimmt, während das andere als zusätzlicher Kanal dient,
- Fig.9: eine teilweise im Längsschnitt gehaltene Anordnung eines am proximalen Ende des Katheters vorgesehenen Schubmanipulators mit Anordnung eines als Bewehrung für den Katheter dienenden Führungsrohres für das Zugelement sowie
- Fig.10: vergrößertem Maßstab den proximalen Teil des Katheters, an welchem die Eintrittsöffnung für den im Inneren des Katheters verlaufenden Kanal an einem seitlichen Stutzen oder dergleichen vorzugsweise mit einem Schleusenventil sichtbar ist.

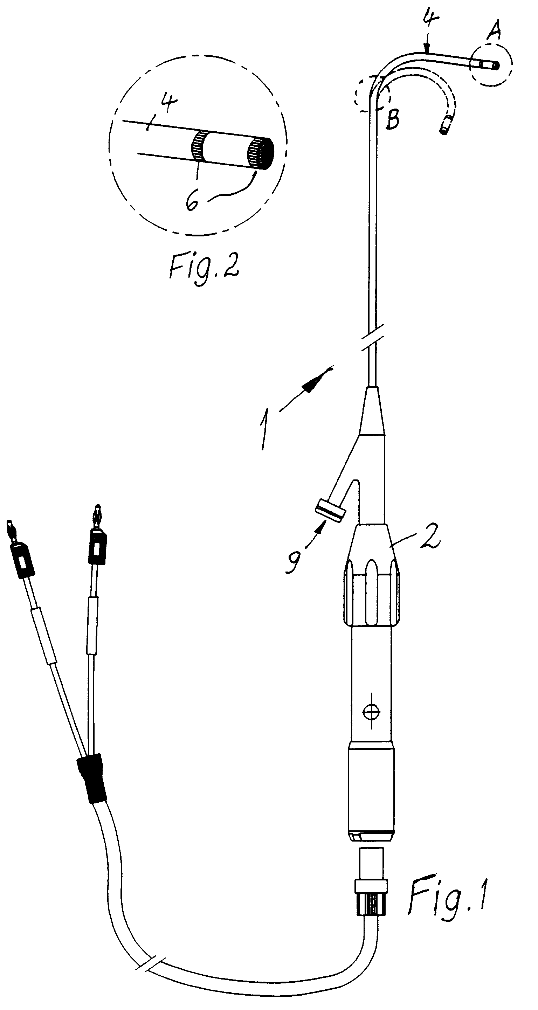

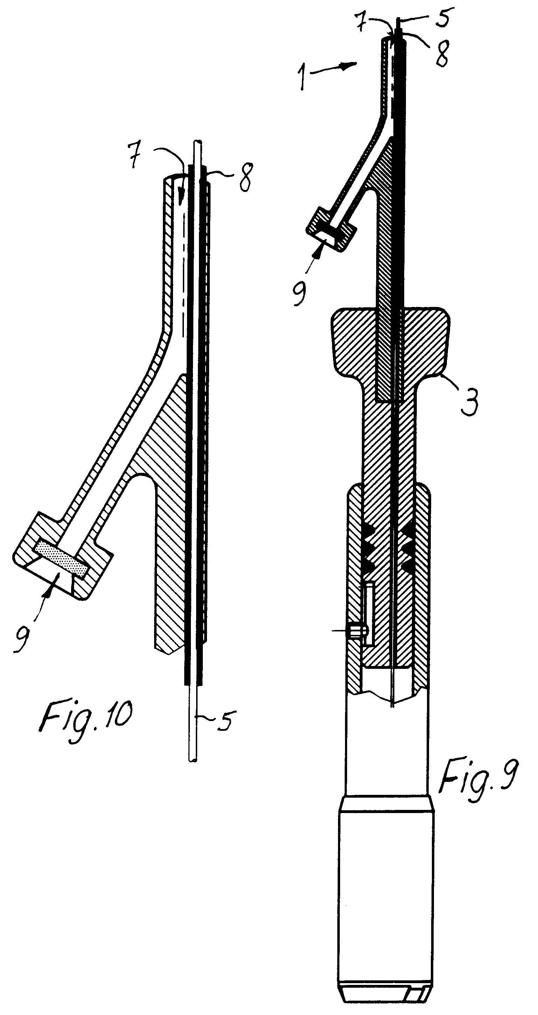

Ein im ganzen mit 1 bezeichneter steuerbarer Katheter weist gemäß Figur 1 einen Drehmanipulator 2 und bei einer abgewandelten Ausführungsform gemäß Figur 9 einen Schubmanipulator 3 auf, womit ein auslenkbarer Bereich 4 mit Hilfe der Manipulatoren und eines im Inneren des Katheters 1 oder Katheterschachtes verlaufenden Zugelementes 5 gemäß Figur 1 mehr oder weniger stark biegbar und dadurch steuerbar ist. Somit können beispielsweise an diesem auslenkbaren Bereich 4 oder der distalen Spitze des Katheters 1 vorgesehene Elektroden 6 in einer gewünschten Weise positioniert werden.

Im Inneren hat der Katheter 1 einen hohlen Kanal 7 (Lumen), durch welchen Instrumente, Therapiegeräte, Endoskope oder dergleichen eingeführt werden können.

Das schon erwähnte Zugelement 5 ist gegenüber der axialen Mitte des Katheters 1 exzentrisch angeordnet, so daß eine auf das Zugelement 5 mit Hilfe eines der Manipulatoren ausgeübten Kraft eine entsprechende Auslenkung des auslenkbaren Bereiches am distalen Ende des Katheters 1 erfolgt. Dadurch läßt sich also die Spitze des Katheters 1 so manipulieren und steuern, daß ein Eintritt in Verzweigungen des Körperhöhlensystemes erleichtert wird. Bis zu dem Auslenkbaren Bereich 4 hin ist das Zugelement 5 in noch zu beschreibender Weise von einer Führung umschlossen.

In allen Ausführungsbeispielen erkennt man, daß der den Katheter 1 bildende Kunststoffschlauch einen stetigen Querschnitt, im Ausführungsbeispiel einen kreisförmigen Querschnitt hat, also kein gegenüber dem eigentlichen Querschnitt seitlich vorstehender Wulst zur Aufnahme des Zugelementes 5 vorhanden ist. Statt einer Kreisform könnte der Querschnitt auch oval oder ellipsenförmig sein.

Das Zugelement 5 und seine Führung verlaufen innerhalb dieses Umrisses des Querschnittes des Katheters 1, wie es in den Figuren dargestellt ist. Damit bei einem Zug auf das Zugelement nur der auslenkbare Bereich 4 und nicht der gesamte Katheter 1 gebogen oder gekrümmt wird, ist bis zu diesem auslenkbaren oder biegsamen Bereich 4 des Katheters hin parallel zu dem Zugelement 5 eine den Katheter 1 gegenüber seinem auslenkbaren Bereich aussteifende Bewehrung ebenfalls innerhalb des Katheterquerschnittes vorgesehen. Vor allem bei einem relativ weichen Werkstoff wird auf diese Weise erreicht, daß bei einem Zug auf das Zugelement 5 nur der auslenkbare nachgiebige Bereich 4 gekrümmt wird, weil die Zugkraft dort auf den geringsten Widerstand stößt. Der übrige Teil des Katheters bleibt jedoch in erwünschter Weise weitgehend unverformt, behält aber gleichzeitig eine genügende Biegsamkeit, um in Körperhöhlen oder Blutgefäße eingeführt werden zu können und dabei auch gewisse Krümmungen und Biegungen mitmachen zu können.

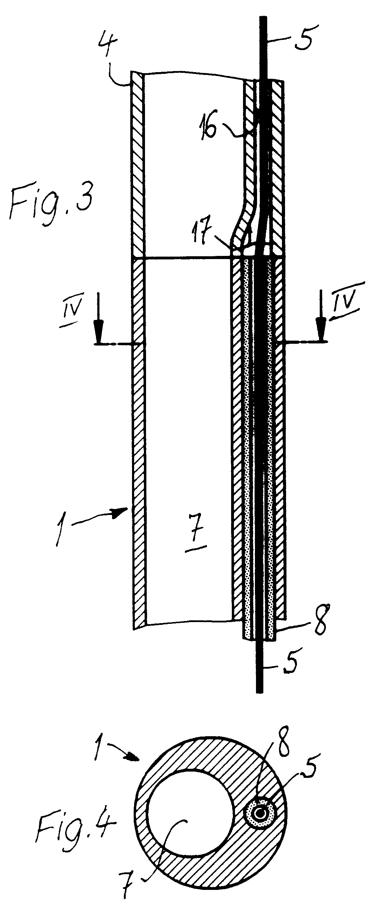

In dem Ausführungsbeispiel gemäß Figur 3 bis 5 sowie 9 und 10 ist die Bewehrung ein das Zugelement 5 in sich aufnehmendes Führungsrohr 8 aus Metall oder hartem Kunststoff, welches innerhalb des Außenquerschnittes des Katheters 1 bis zu dem auslenkbaren Bereich 4 verläuft, wie man es deutlich in Figur 3 erkennt. Gemäß Figur 3 endet das Führungsrohr 8 also am Beginn des auslenkbaren Bereiches 4, wo das zum distalen Ende des auslenkbaren Bereiches 4 hin fortgesetzte Zugelement 5 also aus dem Führungsrohr 8 austritt. Es verläuft dann weiterhin innerhalb des Umrisses des Katheters und dabei des auslenkbaren Bereiches 4 bis zu seiner Befestigungsstelle am distalen Ende dieses auslenkbaren Bereiches 4.

Wird an dem Zugelement 5 eine Zugkraft ausgeübt, wird also aufgrund der exzentrischen Anordnung der auslenkbare Bereich nach der Seiten hin gekrümmt, auf welcher das Zugelement im Inneren dieses auslenkbaren Bereiches 4 verläuft, während gleichzeitig der das Führungsrohr 8 aufweisende Teil des Katheters 1 auch dann weitestgehend unverformt bleibt, wenn der den Katheter 1 bildende Kunststoffschlauch relativ weich ist.

Die Manipulatoren 2 und 3 weisen jeweils eine Eintrittsöffnung 9 auf, die mit dem Lumen oder inneren Kanal 7 des Katheters 1 verbunden ist und durch welche entsprechende Instrument, weitere Katheter, Elektroden, Endoskope oder sonstige Teile oder auch Medikament oder dergleichen eingeführt werden können.

Das das Zugelement in sich aufnehmende Führungsrohr 8 hat einen relativ geringen Außendurchmesser von etwa einem Millimeter oder sogar weniger, eventuell sogar nur von etwa 0,4 Millimeter oder noch darunter und einen Innendurchmesser von etwa 0,2 bis 0,8 Millimeter, also eine Wandstärke von beispielsweise einem Zehntel Millimeter. Dennoch kann es eine genügende Aussteifung des von ihm durchlaufenden Katheters 1 gegen eine Mitverbiegung bewirken, wenn der auslenkbare Bereich durch Betätigung des Zugelementes 5 gekrümmt wird. Andererseits behindert es nicht eine Einführung des Katheters 1, selbst wenn dieser dabei elastisch gebogen wird.

Der Querschnitt oder Durchmesser des Zugelementes 5 kann im Inneren des Führungsrohres mit einem Spiel von etwa einem Hundertstel bis etwa einem Zehntel Millimeter gegenüber dem Innenquerschnitt versehen sein, was für eine leichtgängige Verschiebung des Zugelementes 5 in dem Führungsrohr 8 genügt, unabhängig davon, ob das Zugelement 5 durch einen Manipulator zum krümmen zurückgezogen oder durch die Rückstellkraft des auslenkbaren Bereiches 4 wieder in entgegengesetzter Richtung bewegt wird.

Das Führungsrohr 8 für das Zugelement 5 besteht zweckmäßigerweise aus Edelstahl, um korrosionsfest und unempfindlich gegen Körperflüssigkeiten oder Medikamente zu sein, auch wenn es in die Wandung des Katheters 1 eingebettet ist. Das Zugelement 5 kann ein Draht oder Metallfaden oder auch ein Metallband sein, welches entsprechend flach ist und somit eine Anordnung innerhalb des Katheters 1 mit ganz geringem radialem Platzbedarf erlaubt.

Beim Ausführungsbeispiel gemäß den Figuren 3 bis 5 hat der Kunststoffschlauch, welcher den Katheter 1 im wesentlichen bildet, im Bereich der Unterbringung des Führungsrohres 8 für das Zugelement 5 eine größere Wandstärke als in dem auf einem Durchmesser etwa gegenüberliegenden Wandbereich. Das Führungsrohr 8 ist dabei in diesen verdickten Wandbereich eingebettet. Somit behält der innere Kanal 7 eine regelmäßige, im Ausführungsbeispiel kreisförmige Querschnittsform, wobei allerdings dieser Kanal 7 gegenüber der geometrischen Mitte des Katheters 1 oder des ihn bildenden Schlauches etwas seitlich versetzt ist.

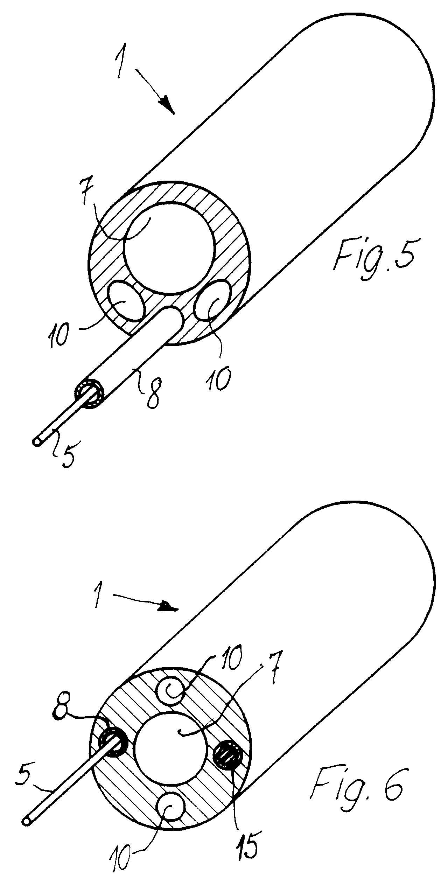

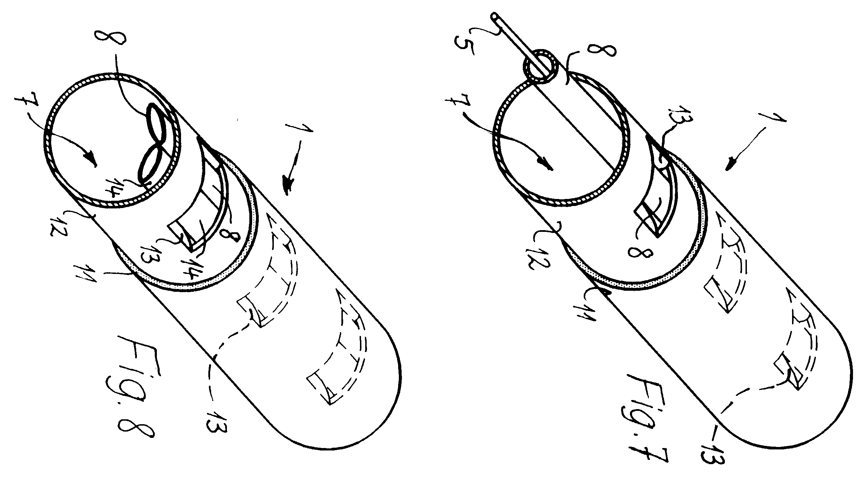

In dem verdickten Wandbereich sind gemäß Figur 5 benachbart zu dem Führungsrohr 8 zwei zusätzliche innere Kanäle 10 mit kleinerem Querschnitt als der eigentliche Hauptkanal 7 angeordnet, um zum Beispiel Drähte für Elektrodenpole oder dergleichen oder Thermistoren und Medikamente führen zu können. Der verdickte Wandbereich zur Einbettung des Führungsrohres 8 kann also zur Unterbringung weiterer, von dem eigentlichen inneren Kanal 7 getrennter Kanäle ausgenutzt werden.

Die Figuren 7 und 8 zeigen eine abgewandelte Ausführungsform des Katheters 1, wobei jeweils nur Abschnitte oder Ausschnitte dieses Katheters 1 in schaubildlicher und geschnittener Darstellung zu sehen sind. Gemäß diesem Ausführungsbeispiel ist der Katheter 1 aus zwei ineinander gesteckten Kunststoffrohren 11 und 12 gebildet, wobei das äußere Rohr 11 die dichte Außenhülle bildet und das innere Rohr 12 in seiner Längsrichtung mit Abstand zueinander in Reihe nebeneinander jeweils radial einwärts gerichtete Klemmfinger, Haltezungen oder Laschen 13 zum Festlegen oder Einklemmen des innenseitig verlaufenden Führungsrohres 8 für das Zugelement 5 aufweist. Diese Laschen 13 können dabei aus diesem innenliegenden Kunststoffrohr 12 teilweise ausgestanzt und in seinen Innenquerschnitt verformt sein.

Gemäß Figur 8 können dabei diese Haltezungen oder Laschen 13 des inneren Rohres 12 zusätzlich zu dem in diesem Falle abgeflachten Führungsrohr 8 ein weiteres Rohr 14 als zusätzlichen Kanal halten. Somit kann auch bei dieser Katheterausbildung ein von dem eigentlichen Lumen oder Kanal 7 unabhängiger weiterer Kanal vorgesehen werden. Gegebenenfalls können dabei die Rohre 8 und 14 auch durch ein Doppelrohr gebildet sein, welches abgeflacht ist.

Eine abgewandelte Art der Aussteifung oder Bewehrung des Katheters 1 ist in Figur 6 dargestellt. Dabei ist nun vorgesehen, daß als zusätzliche Bewehrung parallel zu dem exzentrisch angeordneten Zugelement 5 und Führungsrohr 8 auf der etwa einem Durchmesser gegenüberliegenden Seite ein weiteres Zugelement 15 fest installiert ist, welches bis zu dem Beginn des biegsamen Bereiches 4 verläuft und dort befestigt ist. Wird das bewegliche Zugelement 5 im Sinne einer Krümmung des auslenkbaren Bereiches 4 betätigt, verhindert dieses zusätzliche Zugelement 15 eine Krümmung des nicht auslenkbaren Bereiches des Katheters 1. Es wirkt also gewissermaßen als Gegenzugelement. Es ist also sowohl ein Führungsrohr 8 als auch ein zusätzliches Zugelement 15 als Bewehrungen gemeinsam vorgesehen. Dabei ist in Figur 6 das Gegenzugelement 15 dicker als das bewegliche Zugelement 5 dargestellt; es kann jedoch dieselbe Dicke wie Zugelement 5 haben oder ggf. sogar dünner sein.

In Figur 6 erkennt man, daß der den Katheter 1 in diesem Ausführungsbeispiel bildende Schlauch eine Wand etwa gleichbleibender Dicke hat und an sich auf einem Durchmesser gegenüberliegender Seiten einerseits einen Kanal für das Führungsrohr 8 und das zum Biegen bewegbare Zugelement 5 und andererseits einen Kanal für das unverschiebbare, bis zu dem auslenkbaren Bereich 4 führende und dort endende Zugelement 15, insbesondere einen festen Draht oder ein Band zum Beispiel aus Metall, enthält. Parallel zu diesen Kanälen für Zugelement 5 und Bewehrung 15 sind wiederum weitere Kanäle 10 als Zusatzlumen in der Wandung dieses Katheters 1 angeordnet.

In Figur 3 erkennt man noch, daß das Zugelement 5 in dem auslenkbaren Bereich 4 ebenfalls in einem separaten Kanal 16 jedoch ohne Aussteifung oder Bewehrung geführt ist, der soweit wie möglich von der Mitte des Katheters 1 beziehungsweise des auslenkbaren Bereiches 4 nach außen verlegt ist.

Da das Zugelement 5 bis zu dem bewegbaren Bereich 4 hin in einem Führungsrohr 8 geführt ist - wie Figur 3 es zeigt - ist vorgesehen, daß der Führungskanal 16 für das Zugelement 5 in dem auslenkbaren Bereich 4 des Katheters 1 einen gegenüber dem Führungsrohr 8 radial um dessen Wandstärke nach außen führenden Übergangsbereich 17 hat, damit das Zugelement 5 wirklich so weit wie möglich an die Außenseite dieses bewegbaren Bereiches 4 verlegt werden kann, um unter einem möglichst großen Hebelarm eine Krümmung bewirken zu können. Das Zugelement 5 hat in diesem auslenkbaren Bereich 4 gemäß Figur 3 etwa den Abstand von der Mittelachse des Katheters 1, den die achsferne Mantellinie des Führungsrohres 8 von dieser Mitte hat. Man erkennt in Figur 3, daß das Zugelement in dem Übergangsbereich 17 geringfügig weiter nach außen hin ausgelenkt wird und dann etwa in Verlängerung der außenliegenden Wandung des Führungsrohres 8 weiterverläuft.

Insgesamt ergibt sich ein Katheter 1, der von einem Kunststoffschlauch gebildet ist und eine entsprechende Biegsamkeit und Anpassungsfähigkeit hat, um gut in Körperhöhlensysteme eingeführt werden zu können, insbesondere in Blutgefäße. Dabei kann sein auslenkbarer Bereich 4 mit Hilfe des innenliegenden Zugelementes 5 manipuliert werden, ohne daß das Zugelement 5 an irgend einer Stelle aus dem Katheter 1 oder dem auslenkbaren Bereich 4 austreten muß. Dennoch wird eine Krümmung des nicht auszulenkenden Teiles des Katheters 1 durch eine Bewehrung vermieden, die ein Führungsrohr zum Umschließen des Zugelementes 5 oder ein diesem am Durchmesser gegenüberliegendes weiteres Zugelement 15 sein kann. Somit wird bei der Betätigung des Zugelementes 5 der auslenkbare Bereich 4 um bis zu 180° oder sogar darüber hinaus gekrümmt, ohne daß der übrige Katheterschaft ebenfalls in unerwünschter Weise gekrümmt wird.

Der Katheter 1, welcher von einem biegsamen Kunststoffschlauch gebildet ist, hat in seinem Inneren einen hohlen Kanal 7 und an seinem distalen Ende einen auslenkbaren Bereich 4, um beim Einführen in Abzweigungen beispielsweise von Blutgefäßen gesteuert werden zu können. Zum Auslenken des Bereiches 4 dient ein exzentrisch zur Mitte verlaufendes Zugelement 5, welches von einer eigenen Führung umschlossen ist. Der Katheter 1 hat dabei einen regelmäßigen kreisförmigen, ovalen oder ellipsenförmigen Querschnitt und das Zugelement 5 und seine Führung sind innerhalb des Umrisses dieses Querschnittes untergebracht. Damit der Katheter nur in seinem auslenkbaren Bereich 4 und nicht in dem dazu hinführenden Bereich gekrümmt wird, wenn an dem Zugelement 5 eine Zugkraft aufgebracht wird, weist der Katheter 1 eine bis zu dem auslenkbaren oder biegsamen Bereich 4 etwa parallel zu dem Zugelement 5 verlaufende, den nicht auslenkbaren Bereich aussteifende, aber etwas biegsame Bewehrung ebenfalls innerhalb des Kathederquerschnittes auf. Diese Bewehrung ist ein das Zugelement 5 als Führung in sich aufnehmendes Führungsrohr 8 aus Metall oder hartem Kunststoff und gegebenenfalls ein weiteres Zugelement 15, welches parallel zu dem bewegbaren Zugelement 5 auf einer etwa auf einem Durchmesser gegenüberliegenden Seite des Katheters fest installiert ist und nur bis zu dem auslenkbaren Bereich 4 verläuft.

## Patentansprüche

1. Katheter (1) mit wenigstens einem in seinem Inneren verlaufenden hohlen Kanal (7) oder Lumen, welcher Katheter (1) im wesentlichen von einem biegsamen Kunststoffschlauch gebildet ist, wobei das distale Ende des Katheters (1) mittels eines gegenüber seiner axialen Mitte exzentrisch angeordneten, über die wesentliche Länge des Katheters (1) verlaufenden Zugelementes (5) zum Steuern der Spitzes des Katheters (1) für den Eintritt in Verzweigungen des Körperhöhlensystemes, zum Beispiel Blutgefäße, elastisch biegbar oder auslenkbar ist und wobei das Zugelement (5) distal befestigt und bis zu dem auslenkbaren Bereich (4) von einem eigenen Führungskanal (16) umschlossen ist, wobei das Zugelement (5) bis zu seiner Befestigungsstelle am distalen Ende und sein Führungskanal (16) innerhalb des Umrisses des Querschnittes des Katheters (1) verlaufen, bis zu dem auslenkbaren oder biegsamen Bereich (4) des Katheters (1) parallel zu dem Zugelement (5) eine dem Katheter (1) gegenüber seinem auslenkbaren Bereich aussteifende Bewehrung ebenfalls innerhalb des Katheterquerschnittes vorgesehen ist und der den Katheter (1) bildende Kunststoffschlauch einen ovalen, ellipsen- oder kreisförmigen Querschnitt hat, **dadurch gekennzeichnet,** daß der Führungskanal (16) bis zu dem biegsamen Bereich (4) von einem Führungsrohr (8) gebildet ist, welches bis zu diesem biegsamen Bereich (4) verläuft und dort endet, daß das Führungsrohr (8) als aussteifende Bewehrung vorgesehen ist, daß der Führungskanal (16) für das Zugelement (5) in dem auslenkbaren Bereich (4) einen gegenüber dem Führungsrohr (8) radial etwa um dessen Wandstärke nach außen führenden Übergangsbereich (17) hat und daß das Zugelement (5) in dem auslenkbaren Bereich (4) etwa den Abstand von der Mittelachse des Katheters hat, den die Wandung des Führungsrohres im Bereich ihrer achsfernen Mantellinie hat.

2. Katheter nach Anspruch 1, dadurch gekennzeichnet, daß der Umriß des Querschnittes des Katheters (1) stetig und vorsprungfrei ist.

3. Katheter nach Anspruch 1 oder 2, dadurch gekennzeichnet, daß das Führungsrohr (8) aus Metall, zum Beispiel aus Edelstahl, oder gegebenenfalls aus hartem Kunststoff besteht.

4. Katheter nach einem der Ansprüche 1 bis 3, dadurch gekennzeichnet, daß das das Zugelement (5) in sich aufnehmende Führungsrohr (8) einen Außendurchmesser von etwa einem Drittel oder zwei Fünftel Millimeter bis ungefähr ein Millimeter und einen Innendurchmesser von etwa einem Fünftel bis etwa vier Fünftel Millimeter hat.

5. Katheter nach einem der Ansprüche 1 bis 4, dadurch gekennzeichnet, daß der Querschnitt oder Durchmesser des Zugelementes (5), insbesondere eines Zugdrahtes, Fadens oder Bandes, mit einem Spiel von etwa einem Hundertstel bis etwa einem Zehntel Millimeter gegenüber dem Innenquerschnitt des Führungsrohres (8) versehen ist.

6. Katheter nach Anspruch 5, dadurch gekennzeichnet, daß das Zugelement ein Metallfaden oder Metallband ist.

7. Katheter nach einem der Ansprüche 1 bis 6, dadurch gekennzeichnet, daß der den Katheter (1) bildende Kunststoffschlauch zumindest im Bereich der Anordnung des Führungsrohres (8) für das Zugelement (5) eine größere Wandstärke als in dem auf einem Durchmesser etwa gegenüberliegenden Wandbereich hat und daß das Führungsrohr (8) wenigstens teilweise in den verdickten Wandbereich eingebettet ist.

8. Katheter nach Anspruch 7, dadurch gekennzeichnet, daß in dem verdickten Wandbereich benachbart zu dem Führungsrohr (8) wenigstens ein zusätzlich zu dem inneren Kanal (7) paralleler weiterer Kanal (10) mit insbesondere kleinerem Querschnitt als der des inneren Kanales (7) verläuft.

9. Katheter nach einem der Ansprüche 1 bis 6, dadurch gekennzeichnet, daß der Katheter (1) aus zwei ineinandersteckenden Kunststoffrohren (11, 12) oder Kunststoffschläuchen gebildet ist, wobei das äußere Rohr (11) die dichte Außenhülle bildet, und daß das innere Rohr (12) in seiner Längsrichtung mit Abstand zueinander jeweils radial einwärts gerichtete Klemmfinger, Haltezungen oder Laschen (13) zum Festlegen oder Einklemmen des innenseitig verlaufenden Führungsrohres (8) für das Zugelement (5) aufweist.

10. Katheter nach Anspruch 9, dadurch gekennzeichnet, daß die Haltezungen, Laschen (13) oder dergleichen des inneren Rohres (12) zusätzlich zu dem insbesondere abgeflachten Führungsrohr (8) ein weiteres Rohr (14) als zusätzlichen Kanal halten.

11. Katheter nach einem der Ansprüche 1 bis 10, dadurch gekennzeichnet, daß als eine zusätzliche Bewehrung parallel zu dem exzentrisch angeordneten Zugelement (5) auf der etwa auf einem Durchmesser gegenüberliegenden Seite ein weiteres Zugelement (15) als Gegenzugelement fest installiert ist, welches bis zu dem Beginn des biegsamen Bereiches (4) verläuft und dort sowie am proximalen Ende des Katheters (1) unverschiebbar befestigt ist.

12. Katheter nach Anspruch 11, dadurch gekennzeichnet, daß der ihn bildende Kunststoffschlauch eine Wand etwa gleichbleibender Dicke hat und an sich auf einem Durchmesser gegenüberliegenden Seiten einerseits einen Kanal mit dem Führungsrohr (8) für das zum Biegen relativ zum Katheter (1) bewegbaren Zugelement (5) und andererseits einen Kanal für das unverschiebbare, bis zu dem auslenkbaren Bereich (4) führende und dort endende Zugelement (15), insbesondere einen festen Draht oder ein Band zum Beispiel aus Metall, enthält.

13. Katheter nach Anspruch 11 oder 12, dadurch gekennzeichnet, daß parallel zu den Kanälen für das Zugelement (5) und das als Bewehrung dienendes Gegenzugelement (15) wenigstens ein weitere Kanal (10) als Zusatzlumen in der Wand des Katheters (1) angeordnet ist.

## Claims

1. Catheter (1) having at least one hollow channel (7) or lumen extending inside it, said catheter (1) being formed essentially by a flexible plastics tube, the distal end of the catheter (1) being elastically flexible or deflectable by means of a pulling member (5) which is eccentrically mounted relative to its axial centre and which extends over substantially the entire length of the catheter (1), for controlling the tip of the catheter (1) for insertion into branches in the body cavity system, e.g. blood vessels, the pulling member (5) being distally fixed and surrounded, as far as the deflectable region (4), by its own guide channel (16), whilst the pulling member (5), up to its fixing point at the distal end, and its guide channel (16) extend within the outline of the cross section of the catheter (1), a reinforcement which stiffens the catheter (1) relative to its deflectable region is also provided within the cross section of the catheter, up to the deflectable or flexible region (4) of the catheter (1) parallel to the pulling element (5), and the plastics tube which forms the catheter (1) has an oval, elliptical or circular cross section, characterised in that the guide channel (16), up to the flexible region (4), is formed by a guide tube (8) which runs as far as this flexible region (4) and terminates there, in that the guide tube (8) is provided as a stiffening reinforcement, in that the guide channel (16) for the pulling member (5) has, in the deflectable region (4), a transitional region (17) which leads radially outwards relative to the guide tube (8), approximately by the wall thickness of the latter, and in that the pulling member (5) in the deflectable region (4) is substantially at the same spacing from the central longitudinal axis of the catheter as the wall of the guide tube in the region of its abaxial generatrix.

2. Catheter according to claim 1, characterised in that the outline of the cross section of the catheter (1) is constant and free from projections.

3. Catheter according to claim 1 or 2, characterised in that the guide tube (8) is made of metal, e.g. stainless steel, or optionally hard plastics.

4. Catheter according to one of claims 1 to 3, characterised in that the guide tube (8) which accommodates the pulling member (5) has an external diameter of from about one third or two fifths of a millimetre up to about one millimetre and an internal diameter of from about one fifth to about four fifths of a millimetre.

5. Catheter according to one of claims 1 to 4, characterised in that the cross section or diameter of the pulling member (5), particularly a pulling wire, thread or strip, has a play of from about one hundredth to about one tenth of a millimetre relative to the internal cross section of the guide tube (8).

6. Catheter according to claim 5, characterised in that the pulling element is a metal thread or metal strip.

7. Catheter according to one of claims 1 to 6, characterised in that the plastics tube forming the catheter (1) has a greater wall thickness at least in the region of the mounting of the guide tube (8) for the pulling member (5) than in the wall portion substantially diametrically opposite and in that the guide tube (8) is at least partially embedded in the thickened wall portion.

8. Catheter according to claim 7, characterised in that at least one additional channel (10) parallel to the inner channel (7) with, in particular, a smaller cross section than the inner channel (7) extends in the thickened wall portion adjacent to the guide tube (8).

9. Catheter according to one of claims 1 to 6, characterised in that the catheter (1) is formed by two plastic pipes or tubes (11, 12) fitting one into the other, the outer tube (11) forming the sealed outer shell and the inner tube (12) having radially inwardly directed clamping fingers, retaining tongues or tabs (13) spaced apart from one another in the longitudinal direction of the tube (12), for securing or clamping the inner guide tube (8) for the pulling member (5).

10. Catheter according to claim 9, characterised in that the retaining tongues or tabs (13) or the like of the inner tube (12) hold another tube (14) as an additional channel, in addition to the particularly flattened guide tube (8).

11. Catheter according to one of claims 1 to 10, characterised in that, as an additional reinforcement parallel to the eccentrically mounted pulling member (5), on the side substantially diametrically opposite, another pulling member (15) is fixedly mounted as a counter-pulling member, extending up to the start of the flexible region (4) and immovably secured there and at the proximal end of the catheter (1).

12. Catheter according to claim 11, characterised in that the plastics tube which forms it has a wall of substantially constant thickness and contains on diametrically opposite sides, on the one hand, a channel with the guide tube (8) for the pulling member (5) which is movable relative to the catheter (1) for bending and, on the other hand, a channel for the immovable pulling member (15) which leads to the deflectable region (4) and terminates there, particularly a solid wire or a strip of metal, for example.

13. Catheter according to claim 11 or 12, characterised in that, parallel to the channels for the pulling member (5) and the counter-pulling member (15) serving as a reinforcement, at least one other channel (10) is provided as an additional lumen in the wall of the catheter (1).

## Revendications

1. Cathéter (1) possédant au moins un canal creux (7) ou lumière s'étendant à l'intérieur de lui, cathéter qui est formé essentiellement par un tuyau en plastique flexible, l'extrémité distale du cathéter pouvant être infléchie ou orientée élastiquement au moyen d'un élément de traction (5) disposé de façon excentrée par rapport à son axe et s'étendant sur la partie essentielle de la longueur du cathéter (1), afin de diriger la pointe du cathéter en vue de son entrée dans des ramifications du système de cavités corporelles, des vaisseaux sanguins par exemple, l'élément de traction (5) étant fixé à l'extrémité distale du cathéter et entouré jusqu'à un segment orientable (4) par la paroi d'un canal de guidage (16) qui lui est propre, l'élément de traction (5), jusqu'à son point de fixation à l'extrémité distale, de même que son canal de guidage (16), s'étendant à l'intérieur du contour de la section droite du cathéter, avec prévision, également à l'intérieur de la section droite du cathéter, d'une armature s'étendant jusqu'au segment (4) orientable ou pouvant être infléchi du cathéter, parallèlement à l'élément de traction (5), armature qui raidit le cathéter par rapport à son segment orientable, et le tuyau en plastique formant le cathéter ayant une section droite de forme ovale, elliptique ou circulaire, **caractérisé en ce** que le canal de guidage (16) est formé, jusqu'au segment (4) pouvant être infléchi, par un tube guide (8) qui s'étend jusqu'à ce segment et s'y termine, que le tube guide (8) est prévu comme une armature de raidissement, que le canal de guidage (16) pour l'élément de traction (5) possède, dans le segment orientable (4), une zone de transition (17) qui mène radialement vers l'extérieur, par rapport au tube guide (8), d'une quantité correspondant à peu près à l'épaisseur de paroi de ce tube, et que l'élément de traction (5) dans le segment orientable (4) est espacé de l'axe du cathéter d'une distance correspondant à peu près à la distance entre la génératrice éloignée dudit axe de la paroi du tube guide et cet axe.

2. Cathéter selon la revendication 1, caractérisé en ce que le contour de la section droite du cathéter (1) est continu et exempt de saillie.

3. Cathéter selon la revendication 1 ou 2, caractérisé en ce que le tube guide (8) est en métal, par exemple en acier fin, ou éventuellement en matière plastique dure.

4. Cathéter selon une des revendications 1 à 3, caractérisé en ce que le tube guide (8), recevant l'élément de traction (5), possède un diamètre extérieur allant à peu près d'un tiers ou de deux cinquièmes d'un millimètre jusqu'à environ un millimètre, et un diamètre intérieur allant à peu près d'un cinquième jusqu'à environ quatre cinquièmes d'un millimètre.

5. Cathéter selon une des revendications 1 à 4, caractérisé en ce que la section ou le diamètre de l'élément de traction (5), constitué en particulier par un fil de traction, un filament ou une bande, est pourvu d'un jeu d'environ un centième jusqu'à environ un dixième de millimètre dans la section intérieure du tube guide (8).

6. Cathéter selon la revendication 5, caractérisé en ce que l'élément de traction est un filament métallique ou une bande métallique.

7. Cathéter selon une des revendications 1 à 6, caractérisé en ce que le tuyau en plastique formant le cathéter (1) possède, tout au moins dans la zone où est disposé le tube guide (8) pour l'élément de traction (5), une plus grande épaisseur de paroi que dans une zone de paroi située à peu près diamétralement à l'opposé, et que le tube guide (8) est au moins partiellement noyé dans la zone de paroi épaissie.

8. Cathéter selon la revendication 7, caractérisé en ce qu'au moins un autre canal (10), s'ajoutant au canal intérieur (7) et ayant en particulier une plus petite section que ce canal intérieur, s'étend parallèlement à lui dans la zone de paroi épaissie, à proximité du tube guide (8).

9. Cathéter selon une des revendications 1 à 6, caractérisé en ce que le cathéter (1) est formé de deux tubes en plastique (11, 12) ou de deux tuyaux flexibles en plastique emboîtés l'un dans l'autre, dont le tube extérieur (11) forme l'enveloppe ou gaine extérieure étanche, et que le tube intérieur (12) porte, à distance les uns des autres dans le sens de sa longueur, des doigts de serrage, dès languettes de maintien ou des pattes (13) dirigés radialement vers l'intérieur pour la fixation ou la retenue par serrage du tube guide (8), passant à l'intérieur, de l'élément de traction (5).

10. Cathéter selon la revendication 9, caractérisé en ce que les languettes de maintien, les pattes (13) ou les éléments semblables du tube intérieur (12) maintiennent, en plus du tube guide (8), lequel est notamment aplati, un autre tube (14) formant un canal supplémentaire.

11. Cathéter selon une des revendications 1 à 10, caractérisé en ce qu'un autre élément de traction (15), constituant un élément de traction antagoniste, est installé fixe, en tant qu'armature supplémentaire, parallèlement à l'élément de traction (5) disposé excentriquement, sur le côté du cathéter situé à peu près diamétralement en face, élément de traction supplémentaire qui s'étend jusqu'au début du segment (4) pouvant être infléchi, où il est fixé, un autre point de fixation empêchant le déplacement de cet élément se trouvant à l'extrémité proximale du cathéter (1).

12. Cathéter selon la revendication 11, caractérisé en ce que le tuyau en plastique qui le constitue, possède une paroi dont l'épaisseur est sensiblement constante et contient, sur des côtés qui se font face diamétralement, d'un côté un canal avec un tube guide (8) pour l'élément de traction (5) déplaçable par rapport au cathéter (1) en vue de l'inflexion et, de l'autre côté, un canal pour l'élément de traction (15) qui ne peut pas être déplacé, menant jusqu'au segment orientable (4) et s'y terminant, élément qui est formé notamment par un fil ou une bande fixe, en métal par exemple.

13. Cathéter selon la revendication 11 ou 12, caractérisé en ce qu'au moins un autre canal (10) est ménagé en tant que lumière additionnelle dans la paroi du cathéter (1), parallèlement aux canaux pour l'élément de traction (5) et l'élément de traction antagoniste (15) servant d'armature.
